# EUROPEAN PATENT APPLICATION

(11) **EP 4 331 573 A1**
(43) Date of publication of application: **06.03.2024**
(21) Application number: 23194029.7
(22) Date of filing: 29.08.2023
(51) Int. Cl.: A61K 9/20, A61K 9/28

(54) **TABLET BASED ON AN ESTER OR SALT OF N-BUTYRIC ACID**

(30) Priority: 29.08.2022 IT 202200017748
(71) Applicant: SILA S.P.A., 30033 Noale (VE) (IT)
(72) Inventor: LORENZON, Maurizio, 30033 Noale (VE) (IT)
(74) Representative: Susanetto, Carlo

(57) **Abstract**

Tablet comprising a core (a), in which there is dispersed as the active ingredient an ester or a salt of n-butyric acid, and in which the core (a) has a hardness greater than 200 N.

## Description

### TECHNICAL FIELD

The present invention relates to a tablet, the core of which, in which there is dispersed an ester or a salt of n-butyric acid, has characteristics which make it particularly suitable for being covered with a film.

### PRIOR ART

It is known that some compounds of n-butyric acid, particularly the esters and salts thereof, have advantageous biological effects on the digestive system, stimulating the growth of intestinal villi and modifying the development of the enteric microorganisms for restoring correct intestinal bacterial flora.

Butyric acid is a short chain monocarboxylic fatty acid (with 4 carbon atoms) which is classified among the volatile fatty acids (VFA) together with acetic acid (chain with two carbon atoms) and propionic acid (chain with 3 carbon atoms) which are generally known as SCFA (short chain fatty acids).

Only a minimum percentage of SCFA is introduced with the food product while the greater part is the result of anaerobic fermentation of the undigested fibres present in the food products, in particular cellulose and starch, by means of the bacteria which colonize the colon.

In particular, butyric acid together with propionic acid constitutes approximately from 90% to 95% by weight of the short chain fatty acids which are present inside the large intestine, where the butyric acid constitutes the main source of energy of the colonizing bacteria. Therefore, the lack of this fatty acid in the large intestine constitutes one of the main causes of atrophy of the intestinal mucosa and inflammatory states thereof and increases the risk of developing intestinal tumours, particularly in the colon.

Vice versa, it has been documented that the increase in the values of butyric acid at the enteric level brings about a preventive action and an action of treating some intestinal pathologies, such as colorectal cancer, colitis both of the infective and of the noninfective type, ulcerative colitis, ulcerative rectal colitis, Crohn's disease and irritable bowel syndrome (IBS), as a result of the capacity of butyric acid to stimulate the immune response of the organism and the specific anti-inflammatory action thereof and repairing action of the intestinal mucosa.

In the event of reduced or insufficient endogenous production of butyric acid, the introduction thereof into the diet is therefore recommended, therefore being used in the treatment of the above-mentioned pathologies.

The compounds of n-butyric acid of greatest interest are the salts thereof, which are generally known as "butyrate", and in particular the sodium salt thereof. These compounds, which are generally defined as butyrates, depending on the surrounding environment may be in a dissociated form or in a non-dissociated form, the latter form being the most effective because it is capable of being captured at the cellular level. However, it has been found that the administration of butyric acid and the salts thereof is affected by two types of problems.

The first problem involves the particularly unpleasant odour of rancid butter of this molecule which may cause the patient to interrupt the treatment or the inclusion thereof in the dietary regime.

A second disadvantage is that the compounds of the butyric acid are particularly sensitive to acidic environments, as a result of which, if they are administered per se, they would readily hydrolyze at a gastric level, making the butyrate no longer available for the absorption thereof at an intestinal level.

Therefore, the conventional tablets which contain butyrate, when coming into contact with the gastric environment, would tend to be dissolved in the stomach and thereby be absorbed by the gastrointestinal mucosa before reaching the portion of the intestine where the action thereof is required. Consequently, the quantity of butyrate would be inadequate for bringing about an effective therapeutic or preventive activity of the pathologies mentioned above.

Currently, therefore, there is a need to provide formulations which do not release butyric acid before arriving at the portion of the intestine where the therapeutic activity thereof is required.

There is described in Italian patent application 102018000005908 a tablet which has a gastro-resistant covering on the basis of anionic polyacrylates and in which the core which is constituted to the greatest extent by the active ingredient and by an admixture of microcrystalline cellulose and hydroxypropyl methylcellulose is prepared by dry mixing and subsequent compression.

However, this type of preparation of the core has a number of limitations in the industrial production thereof because the core which is obtained in this manner is not provided with the adequate compactness and hardness necessary during the subsequent steps of processing thereof. In fact, the cores which are prepared with this processing in the dry state readily tend to crumble, for example, in the subsequent covering step which is generally carried out in a coating pan.

Therefore, there is felt the need to provide tablets, the cores of which remain compact and stable in the subsequent processing steps, notwithstanding the fact that they contain high quantities of an ester or salt of the n-butyric acid.

### SUMMARY OF THE INVENTION

The Applicant has now realized tablets, the core (a) of which, in which there is dispersed as an active ingredient an ester or a salt of n-butyric acid, has a high hardness which allows an excellent capacity thereof for processing, particularly during the subsequent steps of covering with a film and handling of the tablet.

Therefore, in a first aspect thereof the present invention is directed to a tablet, the core (a) of which contains:
a) the active ingredient at concentrations between 40% and 80%, preferably between 45% and 75%, even more preferably between 50% and 70% by weight relative to the total weight of the core.

Preferably, the core contains:
b) at least one stabilizing agent comprising or constituted by hydroxypropyl methylcellulose, more preferably with a viscosity between 4 and 15 cP, or polyvinyl pyrrolidone each at concentrations between 3% and 15% by weight relative to the total weight of the core.

Preferably, the core contains:
c) at least one acidity regulator which is more preferably selected from calcium carbonate, calcium hydroxide or relative admixtures at total concentrations between 9% and 20% by weight relative to the total weight of the core.

Preferably, the core contains:
d) at least one filler, more preferably comprising or constituted by sorbitol or microcrystalline cellulose or relative admixtures at total concentrations between 1.5% and 15.5% by weight relative to the total weight of the core.

Preferably, the core contains:
e) at least one binder, more preferably comprising or constituted by corn starch or maltodextrin or relative admixtures at total concentrations between 0.5% and 9% by weight relative to the total weight of the core.

Preferably, the core contains:
f) at least one anti-caking agent, more preferably selected from calcium stearate or magnesium stearate or zinc stearate or silicon dioxide or talcum or relative admixtures at total concentrations between 0.1% and 2.5% by weight relative to the total weight of the core.

Preferably, the core contains:
g) at least one thickening agent, more preferably selected from guar gum or sodium alginate or xanthan gum or carob flour or relative admixtures at concentrations each between 0.01% and 1.0% by weight relative to the total weight of the core. Preferably, if it is used in admixture, this admixture is formed at a maximum by three of the above-mentioned thickening agents.

In one embodiment, the core is constituted by the above-mentioned compounds from a) to g) in the preferred compounds indicated above.

Preferably, the core has a hardness greater than 200 N.

In a second aspect thereof, the present invention relates to a process for the preparation of a core of a tablet based on an ester or salt of n-butyric acid.

Preferably, the process comprises a step I) in which one or more of the following solutions are prepared:
A) aqueous solution with purified water of the thickening agent g) which in a preferred form is constituted by an admixture of guar gum and sodium alginate at a temperature between 75°C and 85°C, preferably at 80°C;
B) aqueous solution, preferably at ambient temperature, with purified water and a portion of the stabilizing agent b) which is present in the final core and which in a preferred form is constituted by hydroxypropyl methylcellulose and in a particularly preferred manner at such quantities as to constitute approximately 1% by weight relative to the total weight of the core (a);
C) aqueous solution with purified water, the filler d) which in a preferred form is constituted by sorbitol and the binder e) which in a preferred form is constituted by corn starch, at a temperature between 55°C and 65°C, preferably 60°C.

Preferably, the process comprises a step II), in which there is mixed the entire quantity of the active ingredient a), any remaining portion of the stabilizing agent b) (for example, hydroxypropyl methylcellulose) which is not used in the step I) for preparing the aqueous solution B) and possibly the acidity regulator c) which in a preferred form is constituted by an admixture of calcium carbonate and calcium hydroxide.

Preferably, the process comprises a step III) in which there is added between 75°C and 85°C, preferably at 80°C, by means of atomization in a fluidized bed, the aqueous solution A) which is prepared in the step I) over a time preferably between 35 and 40 minutes to the admixture resulting from the step II).

Preferably, the process comprises a step IV) in which there is added, preferably at ambient temperature and preferably by means of atomization in a fluidized bed, the solution B) which is prepared in the step I) to the product resulting from the step III). Preferably, the solution B) is added by means of atomization over a time between 45 and 55 minutes.

Preferably, the process comprises a step V) in which there is added at a temperature between 55°C and 65°C, preferably 60°C, by means of atomization in a fluidized bed, the solution C) which is prepared in the step I) to the product resulting from the step IV). Preferably, the solution C) is added by means of atomization over a time between 35 and 40 minutes.

Preferably, the process comprises a step VI) in which there is carried out the drying of the admixture which is obtained in the step V). Preferably, the drying is carried out at a temperature between 55 and 65°C, preferably 60°C, until obtaining a residual humidity of the admixture < 2%.

Preferably, the process comprises a step VII) in which there is mixed in the dry state the product which results from the step VI) with the anti-caking agent f) which in a preferred form is constituted by an admixture of calcium stearate and silicon dioxide.

Preferably, the process comprises a step VIII) in which the admixture resulting from the step VII) is screened in order to eliminate the particles with a diameter greater than 2 mm.

Preferably, the process comprises a step IX) in which the admixture resulting from the preceding step VIII) is compressed.

The core which is thereby obtained can therefore be covered with a suitable film in order to obtain a tablet which is advantageously film-coated. Naturally, if the core is not covered, the core coincides with the tablet.

Preferably, the film is gastro-resistant in order to release the active ingredient in the intestine.

### DETAILED DESCRIPTION OF THE INVENTION

For the purposes of the present invention, the definition "comprising" or "containing" does not exclude the presence of additional components other than those set out after this definition.

For the purposes of the present invention, the definition "constituted by" is intended to be understood to exclude the presence of these additional components.

For the purposes of the present invention, the viscosity of the hydroxypropyl methylcellulose is determined with a capillary viscosimeter in accordance with what is indicated in the monograph which relates to hydroxypropyl methylcellulose of the United States Pharmacopeia USP 43 of 2020. For the purposes of the present invention, the hardness of the tablet is measured by means of a diametral compression test which is carried out by crushing the tablet along the diameter thereof (if the tablet has a circular cross-section) or along the longer axis thereof (if the tablet has an oblong shape).

In particular, the test is carried out by placing the tablet between two jaws which are brought together at a constant speed until causing the tablet to break. The hardness is determined by measuring the force applied to the jaws in order to obtain the breakage of the tablet. An example of a device which is suitable for this measurement is the Durometro Erweka TBH 425 TD (https://www.erweka.com/tablet-hardness-testers/item/tablet-hardness-tester-tbh-425-series.html).

An important aspect of the core to which the present invention relates is the fact that the hydroxypropyl methylcellulose has a low viscosity between 4 and 15 cP, preferably 6 cP, and has a stabilizing function.

Another important aspect of the invention involves the fact that, unlike the process described in the patent application IT102018000005908 which is cited above and which provides for a step of mixing solids in the dry state and compressing the solid admixture obtained, the process of the present invention provides for a step of granulation and covering of the solid admixture comprising or constituted by the active ingredient a), a portion of the stabilizing agent b) which is preferably constituted by hydroxypropyl methylcellulose and the acidity regulator c) which in a preferred form is constituted by the admixture of calcium carbonate and calcium hydroxide which is subjected to three sequential steps of atomization with the atomized aqueous solutions A), B) and C). Another important aspect of this process of the invention involves the atomization speed which is slower than a normal granulation in each step III), IV) and V) so as to allow the complete administration of the three solutions A), B) and C) within a time between 30 and 60 minutes for each step.

According to a preferred form of the tablet to which the present invention relates, in the core (a):
- the active ingredient is sodium butyrate and is contained at concentrations between 40% and 80% by weight, preferably at concentrations of 63.8% by weight relative to the total weight of the core;
- the stabilizing agent b) is hydroxypropyl methylcellulose at concentrations between 10% and 14% by weight relative to the total weight of the core and having a viscosity of 6 cP;
- the acidity regulator c) is constituted by an admixture of calcium carbonate at concentrations between 10% and 14% by weight relative to the total weight of the core and calcium hydroxide at concentrations between 1% and 4% by weight relative to the total weight of the core;
- the filler d) is constituted by sorbitol which is contained at concentrations between 2% and 5% by weight relative to the total weight of the core;
- the binder e) is corn starch at concentrations between 1% and 4% by weight relative to the total weight of the core;
- the anti-caking agent f) is constituted by an admixture of calcium stearate which is contained at concentrations between 0.1% and 1% by weight relative to the total weight of the core and silicon dioxide at concentrations between 0.2% and 0.9% by weight relative to the total weight of the core;
- the thickening agent g) is an admixture of guar gum at concentrations between 0.01% and 0.5% by weight relative to the total weight of the core and sodium alginate at concentrations between 0.01% and 0.1% by weight relative to the total weight of the core.

This core is characterized by having a hardness between 200N and 240N, preferably 230N.

The tablet which contains the core (a) preferably comprises one or more coverings, at least one of which is gastro-resistant, so that the sodium butyrate is released only at an intestinal level where it is more necessary.

Therefore, the gastro-resistant tablets of the invention are suitable for treating and/or preventing inflammatory intestinal states, such as, for example, ulcerative rectal colitis, Crohn's disease and irritable bowel syndrome (IBS).

The following examples of a composition of the core (a) and the preparation thereof and the data relating to hardness are set out by way of example.

### Example 1 - Qualitative/quantitative composition of a core 1 of 1000 mg

**Table 1 - Qualitative/quantitative composition of the core 1**

| **CORE 1** | |
|---|---|
| **Ingredients** | **Quantity (mg)** |
| SILBUTYR^{®} 98 (Sodium Butyrate) | 638 |
| Calcium Carbonate | 100-140 |
| HPMC (AnyCoat-C AN6) | 100-140 |
| Sorbitol | 20-50 |
| Calcium Hydroxide | 10-40 |
| Corn starch | 10-40 |
| Calcium Stearate | 1-10 |
| Silicon dioxide | 2-9 |
| Guar gum | 0.1-5 |
| Sodium Alginate | 0.1-1 |
| **Total** | **1000** |

### Example 2 - Preparation of the core (a) having the qualitative/quantitative composition included in Table 1.

The following solutions are prepared:
Solution A) 33.75 kg of water containing 0.135 kg of guar gum and 0.135 kg of sodium alginate at 80°C.
Solution B) 32.4 kg of water and 2.7 kg of HPMC AnyCoat-C AN6 at ambient temperature.
Solution C) 21.6 kg of water containing 10.26 kg of sorbitol and 7.56 kg of corn starch at 60°C.

There are mixed 172.26 kg of sodium butyrate, 32.4 kg of HPMC AnyCoat-C AN6, 33.75 kg of calcium carbonate and 8.1 kg of calcium hydroxide.

There is added to the admixture obtained in this manner the solution A) at 80°C in atomized form over a time between 35 and 40 minutes.

The solution B) is added at ambient temperature and in an atomized state over a time between 45 and 55 minutes to the admixture which is subjected to the atomization with the solution A).

Finally, there is added to the admixture which is subjected to the atomization with the solution A) and the atomization with the solution B) the solution C) at 60°C over a time between 35 and 40 minutes.

The water is removed from the atomized admixture with the solutions A), B) and C) by bringing the temperature of the air inside the mixer to 60°C until obtaining an admixture having a residual humidity <2%.

There are added to the dried admixture 1.35 kg of silicon dioxide and 1.35 kg of calcium stearate, respectively.

The admixture obtained is screened in order to eliminate the granules with a diameter > 2 mm.

The compression of the dried material is carried out and there are obtained 270,000 tablets of 1 g each.

### Example 3 - The following Tables 2, 3 and 4 set out the qualitative/quantitative compositions of the core 1/A, 1/B and 1/C.

**Table 2 - Qualitative/quantitative composition of the core 1/A**

| **CORE 1/A** | |
|---|---|
| **Ingredients** | **Quantity (% p/p)** |
| SILBUTYR^{®} 98 (Sodium Butyrate) | 53.17 |
| HPMC (AnyCoat-C AN6) | 21.29 |
| Calcium Hydroxide | 12.50 |
| Calcium Carbonate | 7.50 |
| Refined Coconut oil | 5.00 |
| Calcium Stearate | 0.50 |
| Sodium Alginate | 0.04 |
| **Total** | **100** |

The core 1/A is prepared according to the following operating methods.

The following solutions are prepared:
Solution A) 150 g of water and 0.5 g of sodium alginate at 80°C.
Solution B) 60.0 g of refined coconut oil at 40°C.
Solution C) 150 g of water and 10.0 g of HPMC AnyCoat-C AN6 at ambient temperature.

There are mixed 638.0 g of sodium butyrate, 245.5 g of HPMC AnyCoat-C AN6, 90.0 g of calcium carbonate and 150.0 g of calcium hydroxide.

There are added to the admixture which is obtained in this manner 100 g of water in atomized form at ambient temperature and subsequently the solution A) is added at 80°C in atomized form over a time between 10 and 15 minutes.

The solution B) which is atomized at 40°C is added over a time between 5 and 10 minutes to the admixture which is subjected to the atomization with the solution A). Finally, there is added to the admixture which is subjected to the atomization with the solution A) and the atomization with the solution B) the solution C) at ambient temperature over a time between 10 and 15 minutes.

The water is removed from the atomized admixture with the solutions A), B) and C) by bringing the temperature of the air inside the mixer to 60°C until obtaining an admixture having a residual humidity <2%.

There are added to the dried admixture 6.0 g of calcium stearate.

The admixture obtained is screened in order to eliminate the granules with a diameter > 2 mm.

The compression of the dried material is carried out and there are obtained 1000 tablets of 1.2 g each.

**Table 3 - Qualitative/quantitative composition of the core 1/B**

| **CORE 1/B** | |
|---|---|
| **Ingredients** | **Quantity (%p/p)** |
| SILBUTYR^{®} 98 (Sodium Butyrate) | 53.17 |
| HPMC (AnyCoat-C AN15) | 21.29 |
| Calcium Hydroxide | 12.50 |
| Calcium Carbonate | 7.50 |
| Refined Coconut oil | 5.00 |
| Calcium Stearate | 0.50 |
| Sodium Alginate | 0.04 |
| **Total** | **100** |

The core 1/B is prepared according to the following operating methods.

The following solutions are prepared:
Solution A) 150 g of water and 0.5 g of sodium alginate at 80°C.
Solution B) 60.0 g of refined coconut oil at 40°C.
Solution C) 150 g of water and 7.5 g of HPMC AnyCoat-C AN15 at ambient temperature.

There are mixed 638.0 g of sodium butyrate, 248.0 g of HPMC AnyCoat-C AN15, 90.0 g of calcium carbonate and 150.0 g of calcium hydroxide.

There are added to the admixture which is obtained in this manner 100 g of water in atomized form at ambient temperature and subsequently the solution A) is added at 80°C in atomized form over a time between 10 and 15 minutes.

The solution B) which is atomized at 40°C is added over a time between 5 and 10 minutes to the admixture which is subjected to the atomization with the solution A).

Finally, there is added to the admixture which is subjected to the atomization with the solution A) and the atomization with the solution B) the solution C) at ambient temperature over a time between 10 and 15 minutes.

The water is removed from the atomized admixture with the solutions A), B) and C) by bringing the temperature of the air inside the mixer to 60°C until obtaining an admixture having a residual humidity <2%.

There are added to the dried admixture 6.0 g of calcium stearate.

The admixture obtained is screened in order to eliminate the granules with a diameter > 2 mm.

The compression of the dried material is carried out and there are obtained 1000 tablets of 1.2 g each.

**Table 4 - Qualitative/quantitative composition of the core 1/C**

| **CORE 1/C** | |
|---|---|
| **Ingredients** | **Quantity (%p/p)** |
| SILBUTYR^{®} 98 (Sodium Butyrate) | 67.16 |
| HPMC (AnyCoat-C AN6) | 25.58 |
| Sorbitol | 3.68 |
| Corn Starch | 2.53 |
| Calcium Stearate | 0.53 |
| Silicon Dioxide | 0.53 |
| **Total** | **100** |

The core 1/C is prepared according to the following operating methods.

The following solutions are prepared:
Solution A) 70 g of water containing 35.0 g of sorbitol and 24.0 g of corn starch at 60°C.
Solution B) 180 g of water and 15.0 g of HPMC AnyCoat-C AN6 at ambient temperature.

There are mixed 638.0 g of sodium butyrate, 228.0 g of HPMC AnyCoat-C AN6.

There are added to the admixture which is obtained in this manner the solution A) at 60°C in atomized form over a time between 10 and 15 minutes.

The solution B) which is atomized at ambient temperature is added over a time between 18 and 25 minutes to the admixture which is subjected to the atomization with the solution A).

The water is removed from the atomized admixture with the solutions A) and B) by bringing the temperature of the air inside the mixer to 60°C until obtaining an admixture having a residual humidity <2%.

There are added to the dried admixture 5.0 g of silicon dioxide and 5.0 g of calcium stearate.

The admixture obtained is screened in order to eliminate the granules with a diameter > 2 mm.

The compression of the dried material is carried out and there are obtained 1000 tablets of 0.95 g each.

### Example 4 - The following Table 5 sets out the results of tests carried out on the cores 1, 1/A, 1/B and 1/C which show that the results which are better in terms of hardness are obtained with the core 1 according to the present invention.

**Table 5 - Hardness of the cores 1/A, 1/B, 1/C in comparison with the hardness of the core according to the invention**

| **Formulation** | **1/A** | **1/B** | **1/C** | **1** |
|---|---|---|---|---|
| Hardness (N) | 66 | 65 | 159 | 230 |

## Claims

1. A tablet comprising a core (a), in which there is dispersed as the active ingredient an ester or a salt of n-butyric acid or relevant admixtures and which contains or is constituted by:
a) the active ingredient at concentrations between 40% and 80%, preferably between 45% and 75%, even more preferably between 50% and 70% by weight relative to the total weight of the core;
b) a stabilizing agent comprising or constituted by hydroxypropyl methylcellulose with a viscosity between 4 and 15 cP or polyvinyl pyrrolidone each at concentrations between 3% and 15% by weight relative to the total weight of the core;
c) at least one acidity regulator which is selected from calcium carbonate, calcium hydroxide or relevant admixtures at total concentrations between 9 and 20% by weight relative to the total weight of the core;
d) a filler comprising or constituted by sorbitol, microcrystalline cellulose or relevant admixtures at total concentrations between 1.5% and 15.5% by weight relative to the total weight of the core;
e) at least one binder comprising or constituted by corn starch, maltodextrin or relevant admixtures at total concentrations between 0.5% and 9% by weight relative to the total weight of the core;
f) at least one anti-caking agent selected from calcium stearate, magnesium or zinc stearate, silicon dioxide, talcum or relevant admixtures at total concentrations between 0.1% and 2.5% by weight relative to the total weight of the core;
g) at least one thickening agent selected from guar gum, sodium alginate, xanthan gum or carob flour or relevant admixtures at concentrations each between 0.01% and 1.0% by weight relative to the total weight of the core;
the core having a hardness greater than 200 N.

2. A tablet according to claim 1, wherein in the core (a):
- the active ingredient is sodium butyrate and is contained at concentrations between 40% and 80% by weight, preferably at concentrations of 63.8% by weight relative to the total weight of the core,
- the stabilizing agent b) is hydroxypropyl methylcellulose at concentrations between 10% and 14% by weight relative to the total weight of the core having a viscosity of 6 cP;
- the acidity regulator c) is constituted by an admixture of calcium carbonate at concentrations between 10% and 14% by weight relative to the total weight of the core and calcium hydroxide at concentrations between 1% and 4% by weight relative to the total weight of the core;
- the filler d) is constituted by sorbitol which is contained at concentrations between 2% and 5% by weight relative to the total weight of the core;
- the binder e) is corn starch at concentrations between 1% and 4% by weight relative to the total weight of the core;
- the anti-caking agent f) is constituted by an admixture of calcium stearate which is contained at concentrations between 0.1% and 1% by weight relative to the total weight of the core and silicon dioxide at concentrations between 0.2% and 0.9% by weight relative to the total weight of the core;
- the thickening agent g) is an admixture of guar gum at concentrations between 0.01% and 0.5% by weight relative to the total weight of the core and sodium alginate at concentrations between 0.01% and 0.1% by weight relative to the total weight of the core;
and the core has a hardness between 200 and 240 N, preferably 230 N.

3. A process for the preparation of the core (a) of the tablet according to claim 2, comprising the following steps:
I) the following solutions are prepared:
A) aqueous solution with purified water of the thickening agent g) which is constituted by the admixture of guar gum and sodium alginate at a temperature between 75 and 85°C, preferably at 80°C;
B) aqueous solution at ambient temperature with purified water and a portion of the stabilizing agent b) which is constituted by hydroxypropyl methylcellulose at such quantities as to constitute only 1% by weight relative to the total weight of the core (a);
C) aqueous solution with purified water, the filler d) which is constituted by sorbitol and the binder e) which is constituted by corn starch, at a temperature between 55 and 65°C, preferably 60°C;
II) there is mixed the entire quantity of the active ingredient a), the remaining portion of the hydroxypropyl methylcellulose b) which is not used in the step (I) for preparing the aqueous solution B) and the acidity regulator c) which is constituted by the admixture of calcium carbonate and calcium hydroxide;
III) there is added between 75 and 85°C, preferably at 80°C by means of atomization, the aqueous solution A) which is prepared in the step I) over a time between 35 and 40 minutes to the admixture resulting from the step II);
IV) there is added at ambient temperature by means of atomization the solution B) which is prepared in the step I) over a time between 45 and 55 minutes to the product resulting from the step III);
V) there is added at a temperature between 55 and 65°C, preferably 60°C by means of atomization, the solution C) which is prepared in the step I) over a time between 35 and 40 minutes to the product resulting from the step IV);
VI) there is carried out the drying of the admixture which is obtained in the step V) at a temperature between 55 and 65°C, preferably 60°C, until obtaining a residual humidity of the admixture < 2%;
VII) there is mixed in the dry state the product which results from the step VI) with the anti-caking agent f) which is constituted by an admixture of calcium stearate and silicon dioxide,
VIII) the admixture resulting from the step VII) is screened in order to eliminate the particles with a diameter greater than 2 mm;
IX) the admixture resulting from the preceding step is compressed.

4. A tablet according to any one of claims 1 or 2, which is coated with at least one gastro-resistant covering.

5. A gastro-coated tablet according to claim 4, for use in the treatment and/or prevention of intestinal inflammation conditions.

6. A gastro-coated tablet for use according to claim 5, wherein the inflammatory conditions are: ulcerative rectal colitis, Crohn's disease and irritable bowel syndrome (IBS).
